(19) Europäisches Patentamt
European Patent Office
Office européen des brevets

(11) Veröffentlichungsnummer: **0 218 548 B1**

(12) **EUROPÄISCHE PATENTSCHRIFT**

(45) Veröffentlichungstag der Patentschrift: **11.08.93**

(51) Int. Cl.[5]: **C07D 239/91**, C07D 401/04, C07D 413/04, B41M 5/00, G03C 1/76

(21) Anmeldenummer: **86810390.4**

(22) Anmeldetag: **01.09.86**

Die Akte enthält technische Angaben, die nach dem Eingang der Anmeldung eingereicht wurden und die nicht in dieser Patentschrift enthalten sind.

# (54) Chromogene Chinazolinverbindungen.

(30) Priorität: **06.09.85 CH 3855/85**

(43) Veröffentlichungstag der Anmeldung:
**15.04.87 Patentblatt 87/16**

(45) Bekanntmachung des Hinweises auf die Patenterteilung:
**11.08.93 Patentblatt 93/32**

(84) Benannte Vertragsstaaten:
**BE CH DE FR GB IT LI**

(56) Entgegenhaltungen:
**EP-A- 0 033 716**
**EP-A- 0 135 469**

(73) Patentinhaber: **CIBA-GEIGY AG**
**Klybeckstrasse 141**
**CH-4002 Basel(CH)**

(72) Erfinder: **Zink, Rudolf**
**Alemannenstrasse 2**
**CH-4106 Therwil(CH)**
Erfinder: **Fletcher, Ian John, Dr.**
**Bürgenstal 27**
**CH-4312 Magden(CH)**

**EP 0 218 548 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft chromogene Chinazolinverbindungen, Verfahren zu ihrer Herstellung und ihre Verwendung als Farbbildner in druckempfindlichen oder wärmeempfindlichen Aufzeichnungsmaterialien.

Aus der EP-A-33716 sind chromogene Chinazolinverbindungen bekannt, die in 2-Stellung durch eine 4-Aminophenylgruppe substituiert sind und als Substituenten in 4-Stellung des Chinazolinsystems eine Phenoxygruppe oder einen Alkoxyethoxyrest aufweisen. Desweitern werden in der EP-A-135 469 weitere chromogene Chinazolinverbindungen beschrieben, die in 2-Stellung einen heterocyclischen und insbesondere einen Indolinyl-, Tetrahydrochinolyl- oder Benzomorpholinorest enthalten.

Die Chinazolinverbindungen entsprechen der allgemeinen Formel

(1) 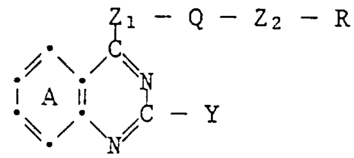

worin

| | |
|---|---|
| R | einen unsubstituierten oder durch Halogen, Cyano, Nitro, Niederalkyl, Niederalkoxy, Niederalkoxycarbonyl oder Niederalkylcarbonyl substituierten Phenyl-, Biphenylyl- oder Naphthylrest, einen unsubstituierten oder durch Halogen, Cyano, Niederalkyl, Niederalkoxy, Niederalkoxycarbonyl und/oder ankondensierten Benzolring substituierten Thienyl-, Furyl-, Pyrrolyl-, Pyrazolyl-, Imidazolyl-, Pyridinyl-, Pyrrolidinyl-, Piperidinyl-, Pipecolinyl-, Morpholinyl-, Thiomorpholinyl-, Piperazinyl- oder N-Methylpiperazinylrest, |
| $Z_1$ | Sauerstoff oder Schwefel, |
| $Z_2$ | Sauerstoff, Schwefel oder -NR', |
| R' | Wasserstoff, unsubstituiertes oder durch Halogen, Cyano oder Niederalkoxy substituiertes Alkyl mit höchstens 12 Kohlenstoffatomen, $C_5$-$C_6$-Cycloalkyl, Phenyl, Benzyl, Phenethyl oder Acyl mit 1 bis 12 Kohlenstoffatomen, oder |
| -NRR' | einen Pyrrolidinyl-, Piperidinyl-, Pipecolinyl-, Morpholinyl-, Thiomorpholinyl-, Piperazinyl- oder N-Methylpiperazinylrest, |
| Q | eine Alkylengruppe mit 2 bis 8 Kohlenstoffatomen, die durch Sauerstoffatome, Schwefelatome oder Iminogruppen-NR'-unterbrochen sein kann, |
| Y | eine Anilin- oder Naphthylaminverbindung die unsubstituiert oder am Ringkohlenstoff durch Halogen, Hydroxyl, Cyano, Nitro, Niederalkyl, Niederalkoxy, Niederalkoxycarbonyl, $C_1$-$C_8$-Alkylcarbonyl, $C_3$-$C_6$-Alkylen, $C_5$-$C_6$-Cycloalkyl, Benzyl oder Phenyl substituiert sein kann und am Stickstoffatom unsubstituiert oder durch ein gegebenenfalls durch Cyano, Halogen, Nitro, Hydroxyl, Niederalkyl, Niederalkoxy oder Niederalkoxycarbonyl substituiertes $C_1$-$C_{12}$-Alkyl, $C_2$-$C_{12}$-Alkenyl, Benzyl oder Phenethyl mono- oder disubstituiert sein kann<br>oder eine Indol-, Carbazol-, Indolin-, Tetrahydrocarbazol-, Dihydrochinolin-, Tetrahydrochinolin-, Dibenzylimid- oder Benzomorpholinoverbindung die über den ankondensierten Benzolring an das Chinazolin in Formel (1) gebunden ist und am Ringkohlenstoff unsubstituiert oder durch Halogen, Hydroxyl, Cyano, Nitro, Niederalkyl, Niederalkoxy, Niederalkoxycarbonyl, $C_1$-$C_8$-Alkylcarbonyl, $C_3$-$C_6$-Alkylen, $C_5$-$C_6$-Cycloalkyl, Benzyl oder Phenyl substituiert sein kann und am Stickstoffatom unsubstituiert oder durch ein gegebenenfalls durch Cyano, Halogen, Nitro, Hydroxyl, Niederalkyl, Niederalkoxy oder Niederalkoxycarbonyl substituiertes $C_1$-$C_{12}$-Alkyl, $C_2$-$C_{12}$-Alkenyl, Benzyl oder Phenethyl substituiert sein kann und |
| der Ring A | unsubstituiert oder durch Halogen, Cyano, Nitro, Niederalkyl, Niederalkoxy oder Niederalkoxycarbonyl substituiert ist, wobei der Ausdruck "Nieder" sich auf solche Gruppen mit 1 bis 5 Kohlenstoffatomen bezieht. |

Niederalkyl und Niederalkoxy stellen bei der Definition der Reste der Chinazolinverbindungen solche Gruppen oder Gruppenbestandteile dar, die 1 bis 5, insbesondere 1 bis 3 Kohlenstoffatome aufweisen, wie z.B. Methyl, Ethyl, n-Propyl, Isopropyl, n-Butyl, sek.-Butyl, tert.-Butyl oder Amyl oder Isoamyl bzw. Methoxy, Ethoxy, Isopropoxy, tert. Butoxy oder tert. Amyloxy.

"Acyl" ist besonders Formyl, Niederalkylcarbonyl, wie z.B. Acetyl oder Propionyl, oder Benzoyl. Weitere Acylreste sind Niederalkylsulfonyl, wie z.B. Methylsulfonyl oder Ethylsulfonyl sowie Phenylsulfonyl.

Halogen bedeutet in Formel (1) und in den nachfolgenden Formeln beispielsweise Fluor, Brom oder vorzugsweise Chlor.

R ist beispielsweise Phenyl, Biphenylyl oder Naphthyl, die durch Halogen, Cyano, Nitro, Niederalkyl, Niederalkoxy, Niederalkoxycarbonyl oder Niederalkylcarbonyl substituiert sein können; vorzugsweise Phenyl, Chlorphenyl, Methoxyphenyl, Tolyl oder Xylyl.

Als heterocyclischer Rest ist R in erster Linie ein Heterocyclus von aromatischem Charakter. Beispiele derartiger Heterocyclen sind Thienyl, Furyl, Pyrrolyl, Pyrazolyl, Imidazolyl oder vorzugsweise Pyridyl. Diese heterocyclischen Reste können unsubstituiert oder durch Halogen, Cyano, Niederalkyl, Niederalkoxy oder Niederalkoxycarbonyl und/oder ankondensierte Benzolringe substituiert sein.

Der heterocyclische Rest R kann auch gesättigt sein, wie z.B. Pyrrolidino, Piperidino, Pipecolino, Morpholino, Thiomorpholino, Piperazino, N-Methylpiperazino. Bevorzugte gesättigte heterocyclische Reste für R sind Pyrrolidino, Piperidino oder Morpholino.

Q stellt insbesondere eine Alkylengruppe dar, die 2 bis 8 Kohlenstoffatome enthalten und geradkettig oder verzweigt sein kann. Die Alkylengruppe weist vorzugsweise 2 bis 4 Kohlenstoffatome auf. Es handelt sich beispielsweise um die $-CH_2-CH_2-CH_2-$,

$$-CH_2-\underset{CH_3}{\underset{|}{C}H}-,$$

$-CH_2-CH_2-CH_2-CH_2-$,

$$-\underset{CH_3}{\underset{|}{C}H}-$$

oder vorzugsweise $-CH_2-CH_2$-Gruppe.

Der aliphatische Kohlenwasserstoffrest Q kann durch Sauerstoffatome, Schwefelatome oder Iminogruppen -NR'- unterbrochen sein. Dabei bedeutet Q vorteilhafterweise den Rest der Formel

$-(CH_2-CH_2O)_m-CH_2CH_2-$ oder

$$-(CH_2\underset{CH_3}{\underset{|}{C}H}O)_s-CH_2-\underset{CH_3}{\underset{|}{C}H}- \quad ,$$

worin m 1 bis 4, besonders 1 oder 2 und s 1 oder 2 sind.

Vorzugsweise bedeutet Q $-CH_2-CH_2-$.

Wenn $Z_2$ -NR' darstellt und R und R' zusammen mit dem sie verbindenden Stickstoffatom einen fünf- oder sechsgliedrigen Heterozyklus bilden, so ist dieser beispielsweise Pyrrolidino, Piperidino, Pipecolino, Piperazino oder vorzugsweise Morpholino.

$Z_1$ und $Z_2$ sind jeweils vorzugsweise Sauerstoff.

Bevorzugte heterocyclische Reste für Y entstammen von Indolinen, Tetrahydrochinolinen und Benzomorpholinen.

Besonders bevorzugt für die Einführung von Y sind N,N-disubstituierte Aniline oder C- und/oder N-substituierte Tetrahydrochinoline.

Die ein- oder mehrkernigen, carbocyclischen oder heterocyclischen Y-Reste können einfach oder mehrfach ringsubstituiert sein. Als C-Substituenten kommen dabei z.B. Halogen, Hydroxyl, Cyano, Nitro, Niederalkyl, Niederalkoxy, Niederalkoxycarbonyl, Acyl mit 1 bis 8 Kohlenstoffatomen, vorzugsweise Niederalkylcarbonyl, $C_3$-$C_6$-Alkylen, $C_5$-$C_6$-Cycloalkyl, Benzyl oder Phenyl in Frage, während N-Substituenten beispielsweise $C_1$-$C_{12}$-Alkyl, $C_2$-$C_{12}$-Alkenyl, Benzyl oder Phenethyl sind, die jeweils auch z.B. durch Cyano, Halogen, Nitro, Hydroxyl, Niederalkyl, Niederalkoxy oder Niederalkoxycarbonyl substituiert sein

können.

Die Alkyl- und Alkenylreste können geradkettig oder verzweigt sein. Beispiele hierfür sind Methyl, Ethyl, n-Propyl, Isopropyl, n-Butyl, t-Butyl, sek.Butyl, Amyl, n-Hexyl, 2-Ethyl-hexyl, Isooctyl, n-Octyl, Decyl oder n-Dodecyl bzw. Vinyl, Allyl, 2-Methylallyl, 2-Ethylallyl, 2-Butenyl oder Octenyl.

Der Ring A ist vorzugsweise nicht weiter substituiert. Falls er Substituenten aufweist, ist er in erster Linie durch Halogen, Cyano, Niederalkyl oder Niederalkoxy, wie z.B. durch Cyano, Chlor, Methyl oder Methoxy mono- oder disubstituiert.

Vorteilhafte chromogene Chinazolin-Verbindungen entsprechen der Formel

(2)

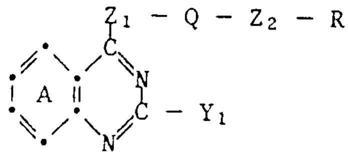

worin A, $Z_1$, $Z_2$, Q und R die angegebene Bedeutung haben und $Y_1$ einen Aminophenylrest der Formel

(2a)

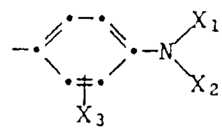

oder einen hydrierten heterocyclischen Rest der Formel

(2b)

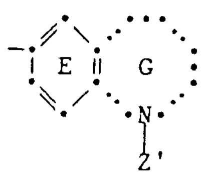

bedeutet, wobei

$X_1$ und $X_2$, unabhängig voneinander, je Wasserstoff, unsubstituiertes oder durch Halogen, Hydroxy, Cyano oder Niederalkoxy substituiertes Alkyl mit höchstens 12 Kohlenstoffatomen, $C_5$-$C_6$-Cycloalkyl, Phenyl, Benzyl, Phenethyl, oder durch Halogen, Nitro, Cyano, Niederalkyl, Niederalkoxy oder Niederalkoxycarbonyl substituiertes Phenyl oder Benzyl

oder $X_1$ und $X_2$ zusammen mit dem sie verbindenden Stickstoffatom einen Pyrrolidinyl- oder Piperidinyl-Rest ünd $X_3$ Wasserstoff, Halogen, Nitro, Niederalkyl, Niederalkoxy oder Niederalkoxycarbonyl und

Z' Wasserstoff oder unsubstituiertes oder durch Halogen, Cyano oder Niederalkoxy substituiertes Alkyl mit höchstens 8 Kohlenstoffatomen, $C_5$-$C_6$-Cycloalkyl, Benzyl oder Phenethyl bedeuten und wobei der Ring E unsubstituiert oder durch Cyano, Halogen, Niederalkyl, wie z.B. Methyl oder Niederalkoxy, wie z.B. Methoxy substituiert sein kann und der Ring G gemeinsam mit dem Ring E einen Indolin-, Tetrahydrochinolin- oder Benzmorpholinrest bedeütet, der unsubstituiert oder durch Halogen, Cyano, Hydroxyl, Niederalkyl, Niederalkoxy, $C_5$-$C_6$-Cycloalkyl, Benzyl oder $C_3$-$C_6$-Alkylen einfach oder mehrfach C-substituiert ist.

Stellen die Substituenten $X_1$ und $X_2$ Alkylgruppen dar, so können sie geradkettig oder verzweigt sein. Beispiele für solche Alkylreste sind Methyl, Ethyl, n-Propyl, Isopropyl, n-Butyl, sek.Butyl, Amyl, n-Hexyl, 2-Ethyl-hexyl, n-Octyl, Isooctyl oder n-Dodecyl.

Sind die Alkylreste in $X_1$ und $X_2$ substituiert, so handelt es sich vor allem um Cyanoalkyl, Halogenalkyl, Hydroxyalkyl oder Alkoxyalkyl mit insgesamt vorzugsweise 2 bis 4 Kohlenstoffatomen, wie z.B. β-Cyanoethyl, β-Chloroethyl, β-Hydroxyethyl, β-Methoxyethyl oder β-Ethoxyethyl.

Beispiele für Cycloalkyl in der Bedeutung von $X_1$ und $X_2$ sind Cyclopentyl oder vorzugsweise Cyclohexyl. Die Cycloalkylreste können einen oder mehrere $C_1$-$C_4$-Alkylreste, vorzugsweise Methylgruppen, enthalten. Vorzugsweise weisen sie insgesamt 5 bis 10 Kohlenstoffatome auf.

Bevorzugte Substituenten in der Benzyl- und Phenylgruppe von $X_1$ und $X_2$ sind z.B. Halogen, Cyano, Methyl, Methoxy oder Carbomethoxy. Beispiele für derartige araliphatische bzw. aromatische Reste sind Methylbenzyl, Chlorbenzyl, Cyanophenyl, Tolyl, Xylyl, Chlorphenyl, Methoxyphenyl oder Carbomethoxyphe-

nyl.

Wenn $X_1$ und $X_2$ zusammen mit dem gemeinsamen Stickstoffstom einen heterocyclischen Rest darstellen, so ist dieser beispielsweise Pyrrolidino, Piperidino, Pipecolino, Morpholino, Thiomorpholino oder Piperazino, wie z.B. N-Methylpiperazino. Bevorzugte heterocyclische Reste für $-NX_1X_2$ sind Pyrrolidino, Piperidino oder Morpholino.

$X_1$ und $X_2$ bedeuten, unabhägnig voneinander, vorzugsweise Niederalkyl, Benzyl, Phenyl, Phenethyl, Niederalkylphenyl oder Niederalkoxyphenyl. $X_3$ bedeutet vorzugsweise Wasserstoff, Chlor, Methyl, Methoxy oder Ethoxy.

Der Ring E ist vorzugsweise unsubstituiert. Er kann jedoch vorteilhafterweise eine Methylgruppe aufweisen. Der Ring G ist vorzugsweise sechsgliedrig und vor allem durch 1, 2 oder 3 Methylgruppen C-substituiert.

Z' ist vorzugsweise Niederalkyl, Benzyl oder $\beta$-Cyanoethyl.

Unter den Chinazolinverbindungen der Formel (2) sind diejenigen, in denen $Y_1$ einen Rest der Formel (2a) darstellt, bevorzugt.

Praktische wichtige chromogene Chinazolin-Verbindungen entsprechen der Formel

(3) 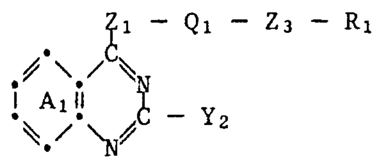

worin

$R_1$ einen gegebenenfalls durch Halogen, Methyl oder Methoxy substituierten Phenyl- oder Naphthylrest,

$Z_1$ Sauerstoff oder Schwefel,

$Z_3$ Sauerstoff, Schwefel, -NH- oder

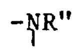

R'' Niederalkyl oder $-NR_1R''$ Morpholino,

$Q_1$ $C_2$-$C_4$-Alkylen oder den Rest

$$-(CH_2-CH_2-O)_{m_1}\,CH_2CH_2-,$$

$Y_2$ einen Aminophenylrest der Formel

(3a) 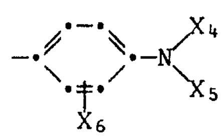 ,

einen 5-Indolinylrest der Formel

(3b) 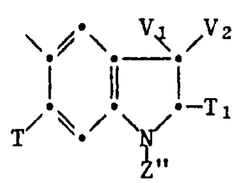 ,

einen Tetrahydrochinolinylrest der Formel

(3c) 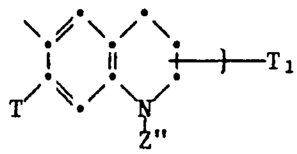 ,

einen Tetrahydrochinolinylrest der Formel

(3d) 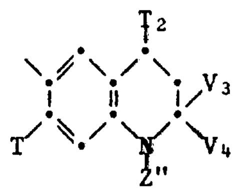

oder einen Benzomorpholinorest der Formel

(3e) 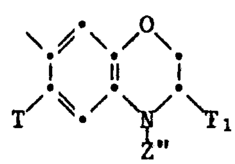

bedeuten, wobei

$m_1$ 1 bis 3

$X_4$ und $X_5$, unabhängig voneinander, Niederalkyl, Cyano-Niederalkyl, Phenyl, Benzyl, Phenethyl, Niederalkylphenyl oder Niederalkoxyphenyl oder

$X_4$ und $X_5$ zusammen mit dem sie verbindenden Stickstoffatom Pyrrolidino, Piperidino oder Morpholino,

$X_5$ Wasserstoff, Halogen, Niederalkyl oder Niederalkoxy,

Z" Wasserstoff, $C_1$-$C_8$-Alkyl, $C_2$-$C_6$-Alkoxyalkyl, $\beta$-Cyanoethyl, Benzyl oder Phenethyl,

T Wasserstoff, Halogen, Niederalkyl, Niederalkoxy, $C_1$-$C_4$-Acylamino, wie z.B. Acetylamino oder Propionylamino, oder Phenyl,

$T_1$ und $T_2$ je Wasserstoff, Halogen, Hydroxy, Niederalkyl oder Niederalkoxy und

$V_1$, $V_2$, $V_3$ und $V_4$ je Wasserstoff, Niederalkyl, $C_5$-$C_6$-Cycloalkyl oder Benzyl oder ($V_1$ und $V_2$) oder ($V_3$ und $V_4$) je zusammen $C_4$-$C_5$-Alkylen bedeuten und

der Ring $A_1$ unsubstituiert oder durch einen oder zwei Substituenten ausgewählt aus Cyano, Halogen, Niederalkyl, Phenyl und Niederalkoxy substituiert sein kann.

Unter den Chinazolinverbindungen der Formel (3) sind diejenigen, in denen $Y_2$ einen Aminophenylrest der Formel (3a) darstellt, bevorzugt. Dabei sind $X_4$ und $X_5$ vorzugsweise Niederalkyl oder Benzyl. $X_5$ ist vorzugsweise Wasserstoff. $R_1$ ist vorzugsweise Phenyl oder Chlorphenyl. $Q_1$ ist insbesondere Ethylen oder Propylen. $Q_1$ ist bevorzugt auch $-CH_2CH_2-O-CH_2CH_2-$. $Z_1$ und $Z_3$ sind vorzugsweise Sauerstoff. Der Ring $A_1$ ist vorzugsweise unsubstituiert.

Bei den Chinazolinverbindungen der Formel (3), in der $Y_2$ einen Rest der Formel (3b), (3c), (3d) oder (3e) darstellt, ist der N-Substituent Z" insbesondere Benzyl, $\beta$-Cyanoethyl oder Alkyl mit 1 bis 8 Kohlenstoffatomen, z.B. n-Octyl, n-Butyl, Isopropyl oder vor allem Methyl oder Ethyl.

Dabei ist

$Y_2$ vorteilhafterweise der Tetrahydrochinolinylrest der Formel (3d) oder vor allem der Benzomorpholinorest der Formel (3e).

T ist vorzugsweise Wasserstoff oder Methyl,

$T_1$ ist vorzugsweise Wasserstoff, Methyl, Hydroxyl oder Chlor.

$T_2$ ist vorzugsweise Wasserstoff, Methyl oder Ethyl.

$V_1$ und $V_2$ bedeuten vorzugsweise Wasserstoff oder Methyl.

$V_3$ und $V_4$ bedeuten vorzugsweise jeweils Niederalkyl und insbesondre jeweils Methyl.

Bedeuten ($V_1$ und $V_2$) oder ($V_3$ und $V_4$) zusammen Alkylen, so weisen sie mit Vorteil 4 oder 5 Kohlenstoffatome auf und bilden mit dem sie verbindenden Kohlenstoffatom einen Cyclopentan- oder Cyclohexanring. Von grossem Interesse sind Chinazolinverbindungen der Formel

(4) 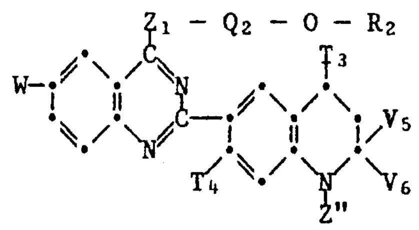

oder vor allem Chinazolinverbindungen der Formel

(5) 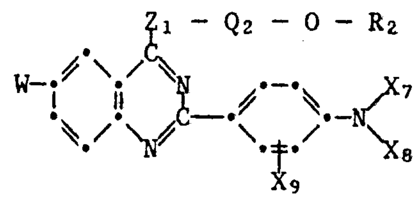

worin $Q_2$ geradkettiges oder verzweigtes Alkylen mit 2 bis 4 Kohlenstoffatomen
$R_2$ Phenyl, Chlorphenyl, Methoxyphenyl, Tolyl oder Naphthyl,
$Z_1$ Schwefel oder vorzugsweise Sauerstoff,
$X_7$ und $X_8$ Niederalkyl oder Benzyl oder $-NX_7X_8$ Piperidino,
$X_9$ Wasserstoff, Methyl, Methoxy oder Ethoxy,
Z" Alkyl mit 1 bis 8 Kohlenstoffatomen, $\beta$-Cyanoethyl oder Benzyl,
$T_3$, $V_5$ und $V_6$ je Niederalkyl, insbesondere Methyl oder Ethyl,
$T_4$ Wasserstoff oder Methyl und
W Halogen, Methyl, Methoxy oder besonders Wasserstoff bedeuten.

Im Vordergrund des Interesses stehen Chinazolinverbindungen der Formel (5), in der $Q_2$ vorzugsweise Propylen oder vor allem Ethylen bedeutet oder auch $-CH_2CH_2-O-CH_2-CH_2-$ darstellt. $R_2$ ist vorzugsweise Phenyl oder Chlorphenyl.

$X_7$ und $X_8$ sind vorzugsweise Benzyl oder vor allem Niederalkyl. W und $X_9$ sind vorzugsweise Wasserstoff.

Die erfindungsgemässen Chinazolinverbindungen der Formel (1) werden dadurch hergestellt, dass man eine Alkohol- bzw. Thioalkoholverbindung der Formel

(6) $HZ_1-Q-Z_2-R$

worin R, Q, $Z_1$ und $Z_2$ die angegebene Bedeutung haben mit einer 4-Halogenchinazolinverbindung der Formel

(7) 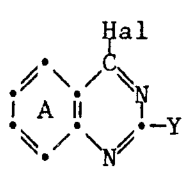

worin A und Y die angegebene Bedeutung haben und Hal Halogen, wie z.B. Brom, Fluor oder vorzugsweise Chlor bedeutet, umsetzt.

Die Umsetzung der Verbindung der Formel (6) mit der Verbindung der Formel (7) erfolgt zweckmässig in Anwesenheit eines säurebindenden Mittels, wie z.B. eines Alkalimetallhydroxides, Alkalimetallcarbonates, einer tertiären Stickstoffbase, wie z.B. Pyridin oder Trialkylaminen und vorzugsweise in Gegenwart auch eines quaternären Ammoniumsalzes, wie z.B. Tetrabutylammoniumbromides, gegebenenfalls in einem

organischen Lösungsmittel oder in einem wässerig-organischen zweiphasigen Medium und bei 60 °C bis zur Rückflusstemperatur.

Als Lösungsmittel kommen beispielsweise cycloaliphatische oder aromatische Kohlenwasserstoffe, wie z.B. Cyclohexan, Benzol, Toluol oder Xylol; Chlorkohlenwasserstoffe, wie z.B. Chloroform, Ethylenchlorid oder Chlorbenzole insbesondere Dichlorbenzol; Ether, wie z.B. Diethylether oder Glykoldimethylether; cyclische Ether, wie z.B. Dioxan oder Tetrahydrofuran; sowie Dimethylformamid, Diethylformamid, Dimethylsulfoxid oder Acetonitril in Betracht.

Alkoholverbindungen der Formel (6), die als Ausgangsstoffe für die Umsetzung mit den Chinazolinverbindungen der Formel (7) eingesetzt werden können, entsprechen vorzugsweise der Formel

(8) HO-Q'-OR,

worin Q' Alkylen mit 2 bis 8 Kohlenstoffatomen bedeutet oder der Formel

(9) $HO-(-CH_2-CH_2-O-)_n-R$

worin n 1 bis 4 und insbesondere 1 oder 2 ist.

Als Beispiele für als Ausgangsstoffe der Formel (6) dienende Alkohole seien genannt: Phenoxyethanol, Chlorphenoxyethanol, Phenoxypropanol, N-β-Hydroxyethl-N-ethylanilin, Phenoxythioethanol, Naphthyloxyethanol, Phenoxydiglykol, Phenoxydithioglykol, Thioethanolanilin, N-(β-Hydroxyethyl)-morpholin.

Die Ausgangsstoffe der Formel (7) können dadurch hergestellt werden, dass man beispielsweise ein 2-Amino-benzoesäureamid der Formel

(10) 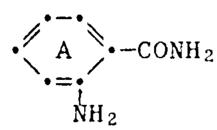

mit einem Aldehyd der Formel

(11) Y-CHO

zu einer 1,2,3,4-Tetrahydro-Chinazolon(4)-Verbindung der Formel

(12) 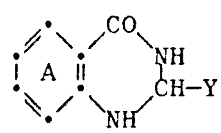

umsetzt, diese zu einer Verbindung der Formel

(13) 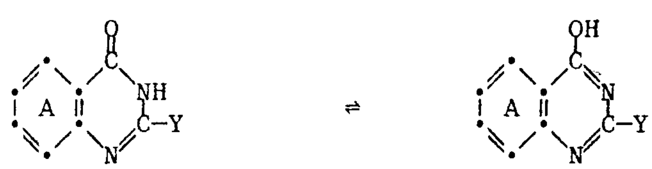

oxidiert, sodann die Hydroxylgruppe am heterocyclischen Ring des Chinazolinsystems durch ein Halogenatom, z.B. mittels Phosphoroxichlorid in Dichlorbenzol oder mittels Thionylchlorid in Dimethylformamid unter Bildung des Ausgangsstoffes der Formel (7) ersetzt. Die erhaltene 4-Halogenchinazolinverbindung kann, ohne isoliert zu werden, weiterverwendet werden.

Die Oxidation der Umsetzungsprodukte der Formel (12) zu den 4-Chinazolon-Verbindungen der Formel (13) erfolgt mit Oxidationsmitteln. Geeignete Oxidationsmittel sind z.B. Chromate, Bichromate, Chlorate,

Chlorite, Peroxide, z.B. Wasserstoffperoxid, Mangandioxid, Bleidioxid, molekularer Sauerstoff, Luft, Perborate, Permanganate, Nitrite, Chlor, Brom und vor allem Chloranil oder Bisulfite.

Die besten Ergebnisse in Bezug auf Ausbeute und Reinheit der erhaltenen 4-Chinazolon-verbindungen erzielt man mit Chloranil als bevorzugtes Oxidationsmittel. Oekologische Vorteile bietet die Oxidation mit Natriumbisulfit. Auf analoge Weise wie in Synthesis 1981, (1), 35 beschrieben, erhält man unter Verwendung dieses Oxidationsmittels Chinazolonverbindungen der Formel (13) in guter Reinheit und Ausbeute.

4-Halogenchinazolinverbindungen der Formel (7) und 4-Chinazolon-Verbindungen der Formel (13) und deren Herstellung werden z.B. in der europäischen Patentveröffentlichung Nr. 33716 beschrieben.

Die Chinazoline der Formeln (1) bis (5) sind normalerweise farblos oder höchstens schwach gefärbt. Wenn diese sublimationsechten Farbbildner mit einem vorzugsweise sauren Entwickler, d.h. einem Elektronenakzeptor, in Kontakt gebracht werden, so ergeben sie intensive gelbe oder orange Farbtöne, die ausgezeichnet lichtecht sind. Sie sind deshalb auch sehr wertvoll im Gemisch mit einem oder mehreren anderen bekannten Farbbildnern, z.B. 3,3-(Bis-aminophenyl)-phthaliden,3-Indolyl-3-aminophenyl-azaphthaliden, 3,3-(Bis-indolyl)-phthaliden, 3-Aminofluoranen, 2,6-Diaminofluoranen, Leukoauraminen, Spiropyranen, Dispiropyranen, Phenoxazinen, Phenothiazinen, Carbazolylmethanen, weiteren Triarylmethanleukofarbstoffen, um blaue, marine-blaue, graue oder schwarze Färbungen zu ergeben.

Die Chinazolinverbindungen der Formeln (1) bis (5) zeigen sowohl auf phenolischen Unterlagen, wie auch besonders auf Tonen und substituierten Zinksalicylaten eine ausgezeichnete Farbintensität und Lichtechtheit. Sie eignen sich vor allem als sehr schnell entwickelnde Farbbildner für die Verwendung in einem wärmeempfindlichen oder insbesondere druckempfindlichen Aufzeichnungsmaterial, das sowohl Kopier- als auch Registriermaterial sein kann. Sie zeichnen sich dadurch aus, dass sie in den Kapselölen hervorragend löslich sind und durch Belichtung im CB-Blatt eine geringe Abnahme der Farbstärke (CB-Desaktivierung) aufweisen. Gegenüber nächstliegenden vorbekannten Chinazolin-Verbindungen zeichnen sie sich dadurch aus, dass sie bedeutend besser sublimationsecht sind.

Ein druckempfindliches Material besteht beispielsweise aus mindestens einem Paar von Blättern, die mindestens einen Farbbildner der Formeln (1) bis (5) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxyd, Aluminiumoxid, Aluminiumsulfat, Aluminiumphosphat, Zinkchlorid, Zinknitrat, Kaolin oder irgendein beliebiger Ton oder sauer reagierende, organische Verbindungen, wie z.B. gegebenenfalls ringsubstituierte Phenole, Salicylsäure oder Salicylsäureester und deren Metallsalze, ferner ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäuteanhydrid mit Styrol, Ethylen oder Vinylmethylether, oder Carboxypolymethylen. Es können auch Mischungen der genannten polymeren Verbindungen eingesetzt werden. Bevorzugte Entwickler sind säureaktiviertes Bentonit, Zinksalicylate oder die Kondensationsprodukte von p-substituierten Phenolen mit Formaldehyd. Die letzteren können auch Zink enthalten.

Die Entwickler können zusätzlich auch mit an sich unreaktiven oder wenig reaktiven Pigmenten oder weiteren Hilfsstoffen wie Kieselgel oder UV-Adsorbern, wie z.B. 2-(2-Hydroxiphenyl)-benzotriazolen gemischt eingesetzt werden. Beispiele für solche Pigmente sind:

Talk, Titandioxid, Zinkoxid, Kreide; Tone wie Kaolin, sowie organische Pigmente, z.B. Harnstoff-Formaldehyd-Kondensate (BET-Oberfläche 2-75 $m^2/g$) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes wird die Farbbildnerlösung auf ein benachbartes, mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. polyhalogeniertes Paraffin oder Diphenyl, wie Chlorparaffin, Monochlordiphenyl oder Trichlordiphenyl, ferner Tricresylphosphat, Di-n-butylphthalat, Dioctylphthalat, Trichlorbenzol, Trichlorethylphosphat, aromatische Ether, wie Benzylphenylether, Kohlenwasserstofföle, wie Paraffin oder Kerosin, z.B. mit Isopropyl, Isobutyl, sek.Butyl

oder tert.Butyl alkylierte Derivate von Diphenyl, Naphthalin oder Terphenyl, Dibenzyltoluol, Terphenyl, partiell hydriertes Terphenyl, mono- bis tetramethylierte Diphenylalkane, benzylierte Xylole, oder weitere chlorierte oder hydrierte, kondensierte, aromatische Kohlenwasserstoffe. Oft werden Mischungen verschiedener Lösungsmittel, insbesondere Mischungen aus Dodecylbenzol, Paraffinölen oder Kerosin und Diisopropylnaphthalin oder partiell hydriertem Terphenyl, eingesetzt, um eine optimale Löslichkeit für die Farbbildner, eine rasche und intensive Färbung und eine für die Mikroverkapselung günstige Viskosität zu erreichen.

Die Kapselwände können durch Koazervationskräfte gleichmässig um die Tröpfchen der Farbbildnerlösung herum gebildet werden, wobei das Einkapselungsmaterial z.B. aus Gelatine und Gummi arabicum bestehen kann, wie dies z.B. in der US-Patentschrift 2 800 457 beschrieben ist. Die Kapseln können vorzugsweise auch aus einem Aminoplast oder modifizierten Aminoplasten durch Polykondensation gebildet werden, wie es in den britischen Patentschriften 989,264, 1 156 725, 1 301 052 und 1 355 124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner der Formeln (1) bis (5) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial. Bevorzugt wird eine Anordnung, bei der der eingekapselte Farbbildner in Form einer Schicht auf der Rückseite eines Uebertragungsblattes und der Elektronenakzeptor in Form einer Schicht auf der Vorderseite eines Empfangsblattes vorhanden sind.

Eine andere Anordnung der Bestandteile besteht darin, dass die den Farbbildner enthaltenden Mikrokapseln und der Entwickler in oder auf dem gleichen Blatt in Form einer oder mehrerer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke oder Stärkederivate oder Polymerlatices. Letzere sind beispielsweise gegebenenfalls carboxylierte Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet.

Die Verbindungen der Formeln (1) bis (5) können auch als Farbbildner in einem thermoreaktiven Aufzeichnungsmaterial verwendet werden. Dieses enthält in der Regel mindestens einen Schichtträger, einen Farbbildner, einen Elektronenakzeptor und gegebenenfalls auch ein Bindemittel und/oder Wachs.

Thermoreaktive Aufzeichnungssysteme umfassen z.B wäremeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet. Die Bilderzeugung (Markierung) kann auch manuell mit einer erhitzten Feder erfolgen. Eine weitere Einrichtung der Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Elektronenakzeptor in Kontakt und es entwickelt sich sofort die erwünschte Farbe.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie sie beispielsweise in der DE-PS 12 51 348 beschrieben sind, z.B. 4-tert.Butylphenol, 4-Phenylphenol, Methylen-bis(p-phenylphenol), 4-Hydroxydiphenylether, $\alpha$-Naphthol, $\beta$-Naphthol, 4-Hydroxy-diphenylsulfon, 4-Hydroxy-4'-methyl-diphenylsulfon, 4-Hydroxybenzoesäuremethylester oder -benzylester, 4-Hydroxyacetophenon, 2,2'-Dihydroxydiphenyl, 4,4'-Isopropylidendiphenol, 4,4'-Isopropyliden-bis-(2-methylphenol), 4,4'-Bis-(hydroxyphenyl)-valeriansäure, Hydrochinon, Pyrogallol, Phloroglucin, p-, m-, o-Hydroxybenzoesäure, Gallussäure, 1-Hydroxy-2-naphthoesäure sowie Borsäure und organische, vorzugsweise aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Chinazolinverbindungen und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, Butadien-styrolcopolymerisate, carboxylierte Butadienstyrolcopolymerisate, Gelatine, Stärke oder veretherte Maisstärke.

Wenn der Farbbildner und der Entwickler in zwei getrennten Schichten vorliegen, können in Wasser unlösliche Bindemittel, d.h. in nicht polaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose oder Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, Titandioxyd, Zinkoxyd, Aluminiumoxid, Aluminiumhydroxyd, Calciumcarbonat, Tone oder auch organische Pigmente, wie z.B. Harnstoff-Formaldehydpolymerisate, enthalten. Um zu bewirken, dass nur innerhalb eines begrenzten Temperaturbereichs die Farbe gebildet wird, können Substanzen, wie Harnstoff, Thioharnstoff, Diphenylthioharnstoff, Acetamid, Acetanilid, Stearinsäureamid, Phthalsäureanhydrid, Metallstearate, wie z.B. Zinkstearate, Phthalsäurenitril, Dimethylterephthalat oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Montanawachs, Paraffinwachs, Polyethylenwachs, Kondensate höherer Fettsäureamide und Formaldehyd und Kondensate höherer Fettsäuren und Ethylendiamin.

Eine weitere Anwendung der Verbindungen der Formeln (1) bis (5) ist die Herstellung eines Farbbildes mittels photohärtbaren Mikrokapseln, wie sie z.B. in der DE-OS 3'247'488 beschrieben wird.

In den folgenden Beispielen beziehen sich die angegebenen Prozentsätze, wenn nichts anderes angegeben ist, auf das Gewicht.

Beispiel 1: 29,3 g der Chinazolonverbindung der Formel

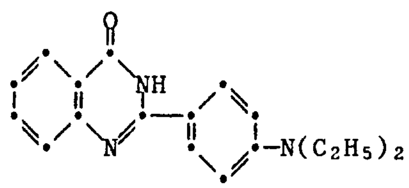

werden in 70 g Toluol bei 85-90 °C mit 16 g Phosphoroxychlorid versetzt. Man rührt die Reaktionsmischung 1 Stunde bei dieser Temperatur und erhält eine dunkelrote Lösung der 4-Chlor-2-(4'-diethylaminophenyl)-chinazolinverbindung der Formel

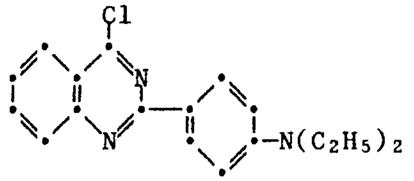

.

Man lässt die Lösung auf eine Suspension von 13,8 g 2-Phenoxyethanol, 10 g Natriumhydroxidlösung (50 %) und 2 g Tetrabutylammoniumbromid zutropfen. Alsdann rührt man die Suspension während 1 1/2 Stunden bei 100-108 °C und versetzt danach bei 90 °C mit 100 ml Wasser. Die Toluolphase wird abgetrennt und mehrmals mit heissem Wasser gewaschen. Das Toluol wird abdestilliert und der Rückstand in 320 g

Methanol eingerührt, wobei man einen kristallinen Niederschlag erhält, welcher bei 15-20 °C abfiltriert, mit Methanol und Wasser gewaschen und getrocknet wird. Man erhält 24 g einer Chinazolin-Verbindung der Formel

(21) 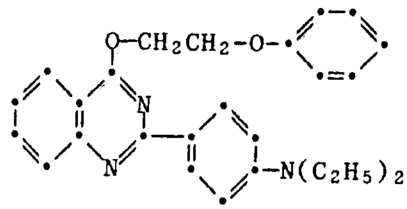

mit einem Schmelzpunkt von 116-119 °C. Auf Säureton entwickelt dieser gut lösliche, sublimierechte Farbbildner eine intensiv grünstichig gelbe Farbe mit guter Lichtechtheit.

Auf gleiche Weise wie in Beispiel 1 beschrieben, erhält man unter Verwendung der entsprechenden Ausgangsstoffe die in der folgenden Tabelle aufgeführten Chinazolinverbindungen der Formel

(22) 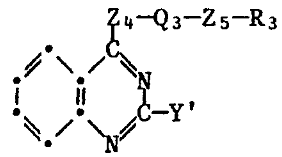

Tabelle

| Beisp. | $Z_4$ | $Q_3$ | $Z_5$ | $R_3$ | Y' | Smp. in °C | Farbe |
|---|---|---|---|---|---|---|---|
| 2 | O | $-CH_2CH_2CH_2-$ | O | 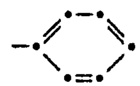 | 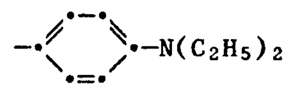 | 107-108 | gelb |
| 3 | O | $-CH_2CH_2-$ | $-N-C_2H_5$ | 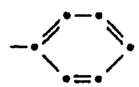 | 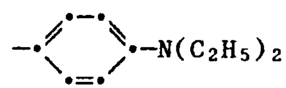 | 90- 92 | gelb |
| 4 | O | $-CH_2CH_2-$ | 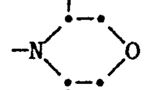 | | 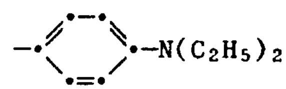 | 84-86 | gelb |
| 5 | O | $-CH_2CH_2-$ | O | 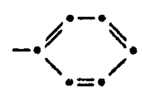 | 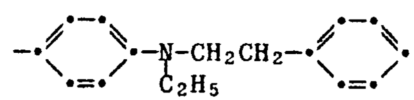 | 114-116 | gelb |
| 6 | O | $-CH_2CH_2-$ | O | 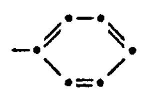 | 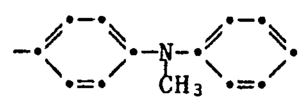 | 140-142 | gelb |

Tabelle (Fortsetzung)

| Beisp. | $Z_4$ | $Q_3$ | $Z_5$ | $R_3$ | Y' | Smp. in °C | Farbe |
|---|---|---|---|---|---|---|---|
| 7 | O | $-CH_2CH_2-$ | O | 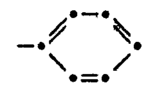 | 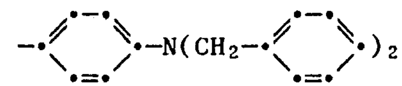 | 104-108 | gelb |
| 8 | S | $-CH_2CH_2-$ | O | 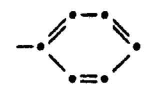 | 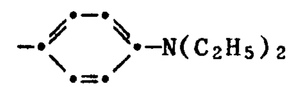 | 90-91 | orange |
| 9 | O | $-CH_2CH_2-$ | O | 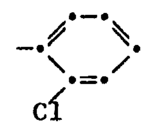 | 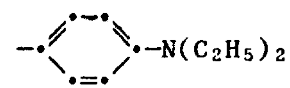 | 109-110 | gelb |
| 10 | O | $-CH_2CH_2-$ | O | 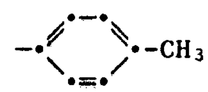 | 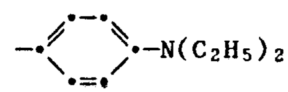 | 104-105 | gelb |
| 11 | O | $-CH_2CH_2-$ | O | 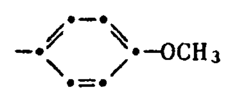 | 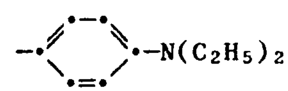 | 109-111 | gelb |
| 12 | O | $-CH_2CH_2-$ | O | 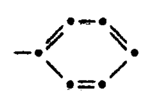 | 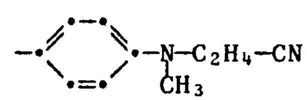 | 163-164 | gelb |

Tabelle (Fortsetzung)

| Beisp. | $Z_4$ | $Q_3$ | $Z_5$ | $R_3$ | Y' | Smp. in °C | Farbe |
|---|---|---|---|---|---|---|---|
| 13 | O | $-CH_2CH_2-$ | O | 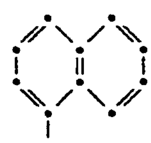 | 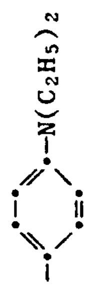 | 146-148 | gelb |
| 14 | O | $-CH_2CH_2-$ | O | 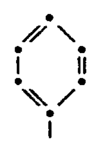 | 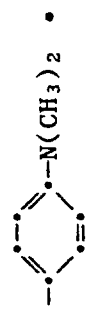 | 154-157 | gelb |
| 15 | O | $-CH_2CH_2-$ | O | 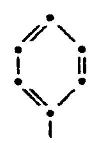 | 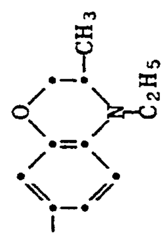 | 111-113 | gelb |

Beispiel 16: Herstellung eines druckempfindlichen Kopierpapiers

Eine Lösung von 3 g der Chinazolinverbindung der Formel (21) in 80 g partiell hydriertem Terphenyl und 17 g Kerosin wird auf an sich bekannte Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit säureaktiviertem Bentonit als Farbentwickler beschichtet. Das erste Blatt und das mit Farbentwickler beschichtete Papier werden mit den Beschichtungen benachbart aufeinandergelegt.

Durch Schreiben mit der Hand oder mit der Schreibmaschine auf dem ersten Blatt wird Druck ausgeübt, und es entwickelt sich sofort auf dem mit dem Entwickler beschichteten Blatt eine intensive gelbe Kopie, die ausgezeichnet lichtecht ist.

Entsprechende intensive, lichtechte gelbe Kopien werden auch bei Verwendung jedes der anderen in den Herstellungsbeispielen 2 bis 15 angegebenen Farbbildner der Formel (22) erzielt.

Beispiel 17: Ersetzt man in Beispiel 16 die Chinazolinverbindung der Formel (21) durch eine Mischung der folgenden Zusammensetzung:
1,4 g 3,3-Bis-(4'-dimethylaminophenyl)-6-dimethylaminophthalid,
1,0 g N-Butylcarbazol-3-yl-bis-(4'-N-methyl-N-phenylaminophenyl)-methan
0,6 g der Chinazolinverbindung der Formel (21)
und 0,5 g 3,3-Bis-(N-n-octyl-2'-methylindol-3-yl)-phthalid
und verfährt im übrigen wie in Beispiel 16 beschrieben, so erhält man ein druckempfindliches Aufzeichnungsmaterial, welches durch Schreiben mit der Hand oder mit der Schreibmaschine eine intensive und lichtechte schwarze Kopie ergibt.

Beispiel 18:

1 g der Chinazolinverbindung der Formel (21) wird in 17 g Toluol gelöst. Zu dieser Lösung gibt man unter Rühren 12 g Polyvinylacetat, 8 g Calciumcarbonat und 2 g Titandioxyd. Die erhaltene Suspension wird im Gewichtsverhältnis 1:1 mit Toluol verdünnt und mit einem 10 µm Rakel auf ein Blatt Papier gestrichen. Auf dieses Blatt Papier wird ein zweites Blatt Papier gelegt, dessen Unterseite bei einem Auftragsgewicht von 3 g $m^2$ mit einer Mischung bestehend aus 1 Teil eines Amidwachses, 1 Teil eines Stearinwachses und 1 Teil Zinkchlorid beschichtet worden ist. Durch Schreiben mit der Hand oder mit der Schreibmaschine auf dem oberen Blatt wird Druck ausgeübt, und es entwickelt sich sofort auf dem mit dem Farbbildner beschichteten Blatt eine intensive, und lichtechte gelbe Farbe.

Beispiel 19: Herstellung eines wärmeempfindlichen Aufzeichnungsmaterials

In einer Kugelmühle werden 32 g 4,4'-Isopropylidendiphenol (Bisphenol A), 3,8 g Distearylamid des Ethylendiamins, 39 g Kaolin, 20 g eines zu 88 % hydrolylsierten Polyvinylalkohols und 500 ml Wasser gemahlen, bis die Teilchengrösse ca. 5 µm beträgt. In einer zweiten Kugelmühle werden 6 g der Verbindung der Formel (21), 3 g eines zu 88 % hydrolysierten Polyvinylalkohols und 60 ml Wasser zu einer Teilchengrösse von ca. 3 µm gemahlen.

Die beiden Dispersionen werden zusammengegeben und mit einem Trockenauftragsgewicht von 5,5 $g/m^2$ auf ein Papier gestrichen. Durch Berührung des Papiers mit einem erhitzten Kugelschreiber wird eine intensive gelbe Farbe erhalten, die eine ausgezeichnete Lichtechtheit hat.

Intensive und lichtechte gelbe Farben können auch bei Verwendung jeder der anderen in den Herstellungsbeispielen 2 bis 15 angegebenen Farbbildner der Formel (22) erhalten werden.

## Patentansprüche

1. Chromogene Chinazolinverbindungen der Formel

(1) 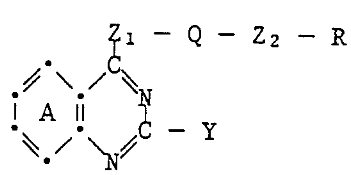

worin

R einen unsubstituierten oder durch Halogen, Cyano, Nitro, Niederalkyl, Niederalkoxy, Niederalkoxycarbonyl oder Niederalkylcarbonyl substituierten Phenyl-, Biphenylyl- oder Naphthylrest, einen unsubstituierten oder durch Halogen, Cyano, Niederalkyl, Niederalkoxy, Niederalkoxycarbonyl und/oder ankondensierten Benzolring substituierten Thienyl-, Furyl-, Pyrrolyl-, Pyrazolyl-, Imidazolyl-, Pyridinyl-, Pyrrolidinyl-, Piperidinyl-, Pipecolinyl-, Morpholinyl-, Thiomorpholinyl-, Piperazinyl- oder N-Methyl-

| | |
|---|---|
| | piperazinylrest, |
| $Z_1$ | Sauerstoff oder Schwefel, |
| $Z_2$ | Sauerstoff, Schwefel oder |

$$-\underset{|}{N}R',$$

| | |
|---|---|
| R' | Wasserstoff, unsubstituiertes oder durch Halogen, Cyano oder Niederalkoxy substituiertes Alkyl mit höchstens 12 Kohlenstoffatomen, $C_5$-$C_6$-Cycloalkyl, Phenyl, Benzyl, Phenethyl oder Acyl mit 1 bis 12 Kohlenstoffatomen, oder |
| -NRR' | einen Pyrrolidinyl-, Piperidinyl-, Pipecolinyl-, Morpholinyl-, Thiomorpholinyl-, Piperazinyl- oder N-Methylpiperazinylrest, |
| Q | eine Alkylengruppe mit 2 bis 8 Kohlenstoffatomen, die durch Sauerstoffatome, Schwefelatome oder Iminogruppen -NR'- unterbrochen sein kann, |
| Y | eine Anilin- oder Naphthylaminverbindung die unsubstituiert oder am Ringkohlenstoff durch Halogen, Hydroxyl, Cyano, Nitro, Niederalkyl, Niederalkoxy, Niederalkoxycarbonyl, $C_1$-$C_8$-Alkylcarbonyl, $C_3$-$C_6$-Alkylen, $C_5$-$C_6$-Cycloalkyl, Benzyl oder Phenyl substituiert sein kann und am Stickstoffatom unsubstituiert oder durch ein gegebenenfalls durch Cyano, Halogen, Nitro, Hydroxyl, Niederalkyl, Niederalkoxy oder Niederalkoxycarbonyl substituiertes $C_1$-$C_{12}$-Alkyl, $C_2$-$C_{12}$-Alkenyl, Benzyl oder Phenethyl mono- oder disubstituiert sein kann oder eine Indol-, Carbazol-, Indolin-, Tetrahydrocarbazol-, Dihydrochinolin-, Tetrahydrochinolin-, Dibenzylimid- oder Benzomorpholinoverbindung die über den ankondensierten Benzolring an das Chinazolin in Formel (1) gebunden ist und am Ringkohlenstoff unsubstituiert oder durch Halogen, Hydroxyl, Cyano, Nitro, Niederalkyl, Niederalkoxy, Niederalkoxycarbonyl, $C_1$-$C_8$-Alkylcarbonyl, $C_3$-$C_6$-Alkylen, $C_5$-$C_6$-Cycloalkyl, Benzyl oder Phenyl substituiert sein kann und am Stickstoffatom unsubstituiert oder durch ein gegebenenfalls durch Cyano, Halogen, Nitro, Hydroxyl, Niederalkyl, Niederalkoxy oder Niederalkoxycarbonyl substituiertes $C_1$-$C_{12}$-Alkyl, $C_2$-$C_{12}$-Alkenyl, Benzyl oder Phenethyl substituiert sein kann und |
| der Ring A | unsubstituiert oder durch Halogen, Cyano, Nitro, Niederalkyl, Niederalkoxy oder Niederalkoxycarbonyl substituiert ist, wobei der Ausdruck "Nieder" sich auf solche Gruppen mit 1 bis 5 Kohlenstoffatomen bezieht. |

**2.** Chinazolinverbindungen gemäss Anspruch 1, dadurch gekennzeichnet, dass in Formel (1) R Phenyl, Biphenylyl oder Naphthyl bedeutet, die jeweils unsubstituiert oder durch Halogen, Cyano, Nitro, Niederalkyl, Niederalkoxy, Niederalkoxycarbonyl oder Niederalkylcarbonyl substituiert sind.

**3.** Chinazolinverbindungen gemäss Anspruch 1, dadurch gekennzeichnet, dass in Formel (1) R Phenyl, Chlorphenyl, Methoxyphenyl, Tolyl oder Xylyl ist.

**4.** Chinazolinverbindungen gemäss einem der Ansprüche 1 bis 3, dadurch gekennzeichnet, dass in Formel (1) Q Alkylen mit 2 bis 4 Kohlenstoffatomen darstellt.

**5.** Chinazolinverbindungen gemäss einem der Ansprüche 1 bis 4, dadurch gekennzeichnet, dass in Formel (1) $Z_1$ und $Z_2$ jeweils Sauerstoff sind.

**6.** Chinazolinverbindung gemäss einem der Ansprüche 1 bis 5, dadurch gekennzeichnet, dass in Formel (1) Y eine am Stickstoffatom durch unsubstituiertes oder durch Cyano, Halogen, Nitro, Hydroxyl, Niederalkyl, Niederalkoxy oder Niederalkoxycarbonyl substituiertes $C_1$-$C_{12}$-Alkyl, $C_2C_{12}$Alkenyl, Benzyl oder Phenethyl, mono- oder disubstituierte Anilin- oder Naphthylaminverbindung darstellt.

**7.** Chinazolinverbindungen gemäss Anspruch 6, dadurch gekennzeichnet, dass in Formel (1) Y den Rest einer N,N-disubstituierten Anilinverbindung darstellt.

**8.** Chinazolinverbindungen gemäss einem der Ansprüche 1 bis 5, dadurch gekennzeichnet, dass in Formel (1) Y den Rest einer N-unsubstituierten oder N-substituierten Indolin-, Tetrahydrocarbazol-, Dihydrochinolin-, Tetrahydrochinolin-, Dibenzylimid- oder Benzomorpholinoverbindung darstellt.

**9.** Chinazolinverbindungen gemäss Anspruch 8, dadurch gekennzeichnet, dass in Formel (1) Y einen Indolinylrest, Tetrahydrochinolinylrest oder Benzomorpholinorest darstellt.

**10.** Chinazolinverbindungen, dadurch gekennzeichnet, dass sie der Formel

(2)

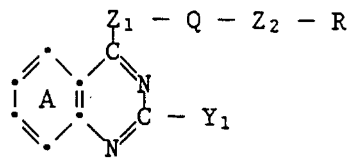

entsprechen, worin A, $Z_1$, $Z_2$, Q und R die im Anspruch 1 angegebene Bedeutung haben und $Y_1$ einen Aminophenylrest der Formel

(2a)

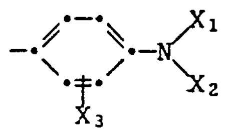

oder
einen hydrierten heterocyclischen Rest der Formel

(2b)

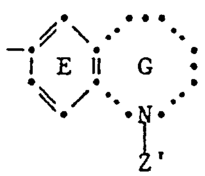

bedeutet, wobei
$X_1$ und $X_2$, unabhängig voneinander, je Wasserstoff, unsubstituiertes oder durch Halogen, Hydroxy, Cyano oder Niederalkoxy substituiertes Alkyl mit höchstens 12 Kohlenstoffatomen, $C_5$-$C_6$-Cycloalkyl, Phenyl, Benzyl, Phenethyl oder durch Halogen, Nitro, Cyano, Niederalkyl, Niederalkoxy oder Niederalkoxycarbonyl substituiertes Phenyl oder Benzyl
oder $X_1$ und $X_2$ zusammen mit dem sie verbindenen Stickstoffatom einen
Pyrrolidinyl- oder Piperidinyl-Rest und
$X_3$ Wasserstoff, Halogen, Nitro, Niederalkyl, Niederalkoxy oder Niederalkoxycarbonyl und
Z' Wasserstoff oder unsubstituiertes oder durch Halogen, Cyano oder Niederalkoxy substituiertes Alkyl mit höchstens 8 Kohlenstoffatomen, $C_5$-$C_6$-Cycloalkyl, Benzyl oder Phenethyl bedeuten und wobei der Ring E unsubstituiert oder durch Cyano, Halogen, Niederalkyl, oder Niederalkoxy substituiert ist und der Ring G gemeinsam mit dem Ring E einen Indolin-, Tetrahydrochinolin- oder Benzmorpholin-Rest, bedeütet der unsubstituiert oder durch Halogen, Cyano, Hydroxyl, Niederalkyl, Niederalkoxy, $C_5$-$C_6$-Cycloalkyl, Benzyl oder $C_3$-$C_6$-Alkylen einfach oder mehrfach C-substituiert ist.

**11.** Chinazolinverbindungen gemäss Anspruch 1, dadurch gekennzeichnet, dass sie der Formel

(3)

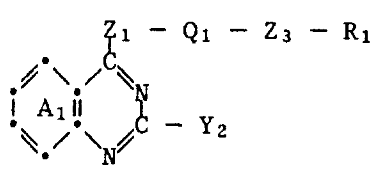

entsprechen, worin
$R_1$ einen gegebenenfalls durch Halogen, Methyl oder Methoxy substituierten Phenyl- oder Naphthylrest,

$Z_1$ Sauerstoff oder Schwefel,
$Z_3$ Sauerstoff, Schwefel, -NH- oder

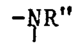

R'' Niederalkyl oder $-NR_1R''$ Morpholino,
$Q_1$ $C_2$-$C_4$-Alkylen oder den Rest

$$-(CH_2-CH_2-O)_{m_1}-CH_2CH_2-$$

$Y_2$ einen Aminophenylrest der Formel

(3a)

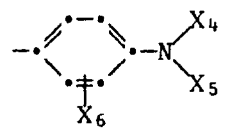

einen 5-Indolinylrest der Formel

(3b)

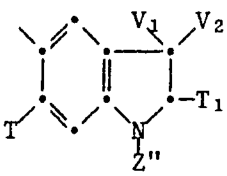

einen Tetrahydrochinolinylrest der Formel

(3c)

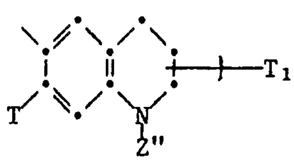

einen Tetrahydrochinolinylrest der Formel

(3d)

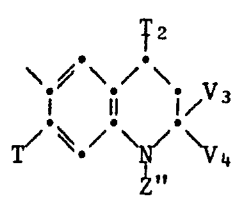

oder einen Benzomorpholinorest der Formel

(3e)

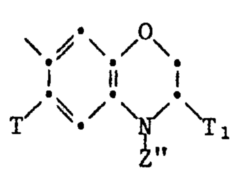

bedeuten, wobei

$m_1$ 1 bis 3
$X_4$ und $X_5$, unabhängig voneinander, Niederalkyl, Cyano-Niederalkyl, Phenyl, Benzyl, Phenethyl, Niederalkylphenyl oder Niederalkoxyphenyl oder
$X_4$ und $X_5$ zusammen mit dem sie verbindenden Stickstoffatom Pyrrolidino, Piperidino oder Morpholino,
$X_5$ Wasserstoff, Halogen, Niederalkyl oder Niederalkoxy,
Z" Wasserstoff, $C_1$-$C_8$-Alkyl, $C_2$-$C_6$-Alkoxyalkyl, $\beta$-Cyanoethyl, Benzyl oder Phenethyl,
T Wasserstoff, Halogen, Niederalkyl, Niederalkoxy, $C_1$-$C_4$-Acylamino oder Phenyl,
$T_1$ und $T_2$ je Wasserstoff, Halogen, Hydroxy, Niederalkyl oder Niederalkoxy und
$V_1$, $V_2$, $V_3$ und $V_4$ je Wasserstoff, Niederalkyl, $C_5$-$C_6$-Cycloalkyl oder Benzyl oder ($V_1$ und $V_2$) oder ($V_3$ und $V_4$) je zusammen $C_4$-$C_5$-Alkylen bedeuten und
der Ring $A_1$ unsubstituiert oder durch einen oder zwei Substituenten ausgewählt aus Cyano, Halogen, Niederalkyl, Phenyl und Niederalkoxy substituiert ist.

**12.** Chinazolinverbindungen gemäss Anspruch 11, dadurch gekennzeichnet, dass in Formel (3) $Y_2$ einen Aminophenylrest der Formel (3a) darstellt.

**13.** Chinazolinverbindungen gemäss Anspruch 12, dadurch gekennzeichnet, dass in Formel (3) $X_4$ und $X_5$ je Niederalkyl oder Benzyl $X_5$ Wasserstoff, $R_1$ Phenyl oder Chlorphenyl, $Q_1$ Ethylen oder Propylen und $Z_1$ und $Z_2$ Sauerstoff bedeuten und der Ring $A_1$ unsubstituiert ist.

**14.** Chinazolinverbindungen gemäss Anspruch 1, dadurch gekennzeichnet, dass sie der Formel

(4)

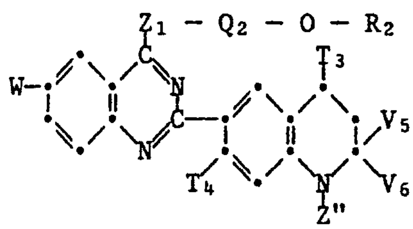

entsprechen, worin

| | |
|---|---|
| $Q_2$ | geradkettiges oder verzweigtes Alkylen mit 2 bis 4 Kohlenstoffatomen |
| $R_2$ | Phenyl, Chlorphenyl, Tolyl, Methoxyphenyl oder Naphthyl, |
| $Z_1$ | Schwefel oder Sauerstoff, |
| Z" | Alkyl mit 1 bis 8 Kohlenstoffatomen, $\beta$-Cyanoethyl oder Benzyl, |
| $T_3$, $V_5$ und $V_6$ | je Niederalkyl, |
| $T_4$ | Wasserstoff oder Methyl und |
| W | Halogen, Methyl, Methoxy oder Wasserstoff bedeuten. |

**15.** Chinazolinverbindungen gemäss Anspruch 1, dadurch gekennzeichnet, dass sie der Formel

(5)

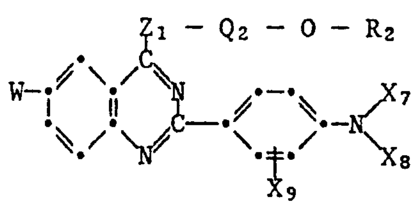

entsprechen, worin

| | |
|---|---|
| $Q_2$ | geradkettiges oder verzweigtes Alkylen mit 2 bis 4 Kohlenstoffatomen |
| $R_2$ | Phenyl, Chlorphenyl, Tolyl, Methoxyphenyl oder Naphthyl, |
| $Z_1$ | Schwefel oder Sauerstoff, |
| $X_7$ und $X_5$ | je Niederalkyl oder Benzyl oder -$NX_7X_5$ Piperidino, |
| $X_9$ | Wasserstoff, Methyl, Methoxy oder Ethoxy und |
| W | Halogen, Methyl, Methoxy oder Wasserstoff bedeuten. |

**16.** Chinazolinverbindungen gemäss Anspruch 15, dadurch gekennzeichnet, dass in Formel (5) $Q_2$ Ethylen oder Propylen, $R_2$ Phenyl oder Chlorphenyl, $X_7$ und $X_8$ Niederalkyl, $X_9$ und W je Wasserstoff bedeuten.

**17.** Verfahren zur Herstellung von Chinazolinverbindungen der im Anspruch 1 angegebenen Formel (1), dadurch gekennzeichnet, dass man eine Alkohol- bzw. Thioalkoholverbindung der Formel

(6) $HZ_1\text{-}Q\text{-}Z_2\text{-}R$

worin R, Q, $Z_1$ und $Z_2$ die in Anspruch 1 angegebene Bedeutung haben mit einer 4-Halogenchinazolinverbindung der Formel

(7)

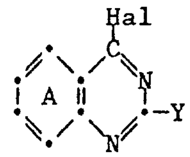

worin A und Y die in Anspruch 1 angegebene Bedeutung haben und Hal Halogen bedeutet, umsetzt.

**18.** Verwendung einer Chinazolinverbindung der in einem der Ansprüche 1 bis 16 angegebenen Formel als Farbbildner in einem druckempfindlichen oder wärmeempfindlichen Aufzeichnungsmaterial.

**19.** Druck- oder wärmeempfindliches Aufzeichnungsmaterial, dadurch gekennzeichnet, dass es in seinem Farbreaktantensystem als Farbbildner mindestens eine Chinazolinverbindung der in einem der Ansprüche 1 bis 16 angegebenen Formel enthält.

**20.** Druckempfindliches Aufzeichnungsmaterial gemäss Anspruch 19, dadurch gekennzeichnet, dass es die Chinazolinverbindung, gelöst in einem organischen Lösungsmittel, und mindestens einen festen Elektronenakzeptor enthält.

**21.** Druckempfindliches Aufzeichnungsmaterial gemäss einem der Ansprüche 19 und 20, dadurch gekennzeichnet, dass die Chinazolinverbindung in Mikrokapseln eingekapselt ist.

**22.** Druckempfindliches Aufzeichnungsmaterial gemäss Anspruch 21, dadurch gekennzeichnet, dass die eingekapselte Chinazolinverbindung in Form einer Schicht auf der Rückseite eines Uebertragungsblattes und der Elektronenakzeptor in Form einer Schicht auf der Vorderseite des Empfangsblattes vorhanden sind.

**23.** Druckempfindliches Aufzeichnungsmaterial gemäss einem der Ansprüche 19 bis 22, dadurch gekennzeichnet, dass die Chinazolinverbindung gemeinsam mit einem oder mehreren anderen Farbbildnern enthalten ist.

**24.** Wärmeempfindliches Aufzeichnungsmaterial gemäss Anspruch 19, dadurch gekennzeichnet, dass es in mindestens einer Schicht mindestens einen Farbbildner, einen Elektronenakzeptor und gegebenenfalls ein Bindemittel enthält, worin der Farbbildner die in einem der Ansprüche 1 bis 16 angegebenen Formel hat.

## Claims

**1.** A chromogenic quinazoline compound of formula

(1)

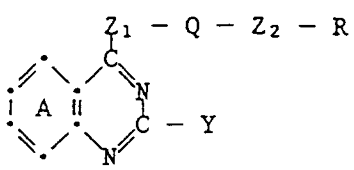

wherein

R is a phenyl, biphenylyl or naphthyl radical, which is unsubstituted or substituted by halogen, cyano, nitro, lower alkyl, lower alkoxy, lower alkoxycarbonyl or lower alkylcarbonyl, or is a thienyl, furyl, pyrrolyl, pyrazolyl, imidazolyl, pyridyl, pyrrolidinyl, piperidinyl, pipecolinyl, morpholinyl, thiomorpholinyl, piperazinyl or N-methylpiperazinyl radical, which is unsubstituted or substituted by halogen, cyano, lower alkyl, lower alkoxy or lower alkoxycarbonyl and/or has a fused-on benzene ring,

$Z_1$ is oxygen or sulfur,

$Z_2$ is oxygen, sulfur or -NR',

R' is hydrogen, alkyl of not more than 12 carbon atoms which is unsubstituted or substituted by halogen, cyano or lower alkoxy, or is $C_5$-$C_6$-cycloalkyl, phenyl, benzyl, phenethyl or acyl of 1 to 12 carbon atoms, or

-NRR'is a pyrrolidinyl, piperidinyl, pipecolinyl, morpholinyl, thiomorpholinyl, piperazinyl or N-methylpiperazinyl radical,

Q is an alkylene group of 2 to 8 carbon atoms, which may be interrupted by oxygen atoms, sulfur atoms or imino groups -NR'-,

Y is an aniline or naphthylamine compound which may be unsubstituted or substituted on the ring carbon by halogen, hydroxyl, cyano, nitro, lower alkyl, lower alkoxy, lower alkoxycarbonyl, $C_1$-$C_8$alkylcarbonyl, $C_3$-$C_6$alkylene, $C_5$-$C_6$cycloalkyl, benzyl or phenyl and on the nitrogen atom may be unsubstituted or mono- or disubstituted by $C_1$-$C_{12}$alkyl, $C_2$-$C_{12}$alkenyl, benzyl or phenethyl which may be substituted by cyano, halogen, nitro, hydroxyl, lower alkyl, lower alkoxy or lower alkoxycarbonyl

or is an indole, carbazole, indoline, tetrahydrocarbazole, dihydroquinoline, tetrahydroquinoline, dibenzylimide or benzomorpholino compound which is attached to the quinazoline in formula (1) via the fused-on benzene ring and on the ring carbon may be unsubstituted or substituted by halogen, hydroxyl, cyano, nitro, lower alkyl, lower alkoxy, lower alkoxycarbonyl, $C_1$-$C_8$alkylcarbonyl, $C_3$-$C_6$-alkylene, $C_5$-$C_6$cycloalkyl, benzyl or phenyl and on the nitrogen atom may be unsubstituted or substituted by $C_1$-$C_{12}$alkyl, $C_2$-$C_{12}$alkenyl, benzyl or phenethyl which may be substituted by cyano, halogen, nitro, hydroxyl, lower alkyl, lower alkoxy or lower alkoxycarbonyl, and

the ring A is unsubstituted or substituted by halogen, cyano, nitro, lower alkyl, lower alkoxy or lower alkoxycarbonyl, the expression "lower" relating to those groups with 1 to 5 carbon atoms.

2. A quinazoline compound according to claim 1, wherein in formula (1) R is phenyl, biphenylyl or naphthyl, each unsubstituted or substituted by halogen, cyano, nitro, lower alkyl, lower alkoxy, lower alkoxycarbonyl or lower alkylcarbonyl.

3. A quinazoline compound according to claim 1, wherein in formula (1) R is phenyl, chlorophenyl, methoxyphenyl, tolyl or xylyl.

4. A quinazoline compound according to one of claims 1 to 3, wherein in formula (1) Q is alkylene of 2 to 4 carbon atoms.

5. A quinazoline compound according to one of claims 1 to 4, wherein in formula (1) $Z_1$ and $Z_2$ are each oxygen.

6. A quinazoline compound according to one of claims 1 to 5, wherein in formula (1) Y is an aniline or naphthylamine compound which is mono- or disubstituted on the nitrogen atom by $C_1$-$C_{12}$alkyl, $C_2$-$C_{12}$alkenyl, benzyl or phenethyl which is unsubstituted or substituted by cyano, halogen, nitro, hydroxyl, lower alkyl, lower alkoxy or lower alkoxycarbonyl.

7. A quinazoline compound according to claim 6, wherein in formula (1) Y is the radical of an N,N-disubstituted aniline compound.

8. A quinazoline compound according to one of claims 1 to 5, wherein in formula (1) Y is the radical of an N-unsubstituted or N-substituted indoline, tetrahydrocarbazole, dihydroquinoline, tetrahydroquinoline, dibenzylimide or benzomorpholino compound.

9. A quinazoline compound according to claim 8, wherein in formula (1) Y is an indolinyl, tetrahydroquinolinyl or benzomorpholino radical.

**10.** A quinazoline compound of formula

(2) 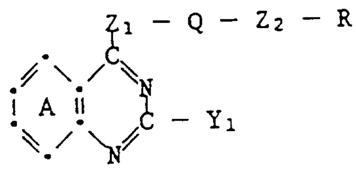

in which A, $Z_1$, $Z_2$, Q and R are as defined in claim 1 and $Y_1$ is an aminophenyl radical of formula

(2a) 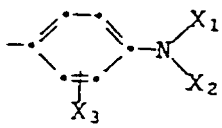

or a hydrogenated heterocyclic radical of formula

(2b) 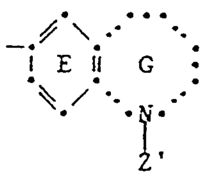

where

$X_1$ and $X_2$ are each independently of the other hydrogen, alkyl of not more than 12 carbon atoms which is unsubstituted or substituted by halogen, hydroxyl, cyano or lower alkoxy, or are $C_5$-$C_6$cycloalkyl, phenyl, benzyl, phenethyl, or phenyl or benzyl substituted by halogen, nitro, cyano, lower alkyl, lower alkoxy or lower alkoxycarbonyl,

or $X_1$ and $X_2$, together with the nitrogen atom which connects them, are a pyrrolidinyl or piperidinyl radical, and

$X_3$ is hydrogen, halogen, nitro, lower alkyl, lower alkoxy or lower alkoxycarbonyl, and

Z' is hydrogen or alkyl of not more than 8 carbon atoms which is unsubstituted or substituted by halogen, cyano or lower alkoxy, or is $C_5$-$C_6$cycloalkyl, benzyl or phenethyl, and the ring E is unsubstituted or substituted by cyano, halogen, lower alkyl or lower alkoxy and the ring G together with the ring E is an indoline, tetrahydroquinoline or benzomorpholine radical which is unsubstituted or C-substituted one or more times by halogen, cyano, hydroxyl, lower alkyl, lower alkoxy, $C_5$-$C_6$cycloalkyl, benzyl or $C_3$-$C_6$alkylene.

**11.** A quinazoline compound according to claim 1, of formula

(3) 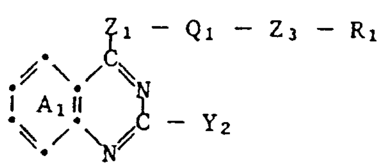

wherein

$R_1$ is a phenyl or naphthyl radical, which is unsubstituted or substituted by halogen, methyl or methoxy,

$Z_1$ is oxygen or sulfur,

$Z_3$ is oxygen, sulfur, -NH- or -NR'',

R'' is lower alkyl or $-NR_1R''$ is morpholino,

$Q_1$ is $C_2$-$C_4$alkylene or the radical $-(CH_2-CH_2-O)m_1-CH_2CH_2-$,

$Y_2$ is an aminophenyl radical of formula

(3a) 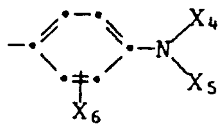

or a 5-indolinyl radical of formula

(3b) 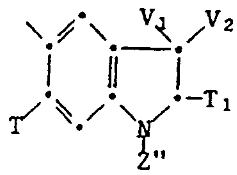

or a tetrahydroquinolinyl radical of formula

(3c) 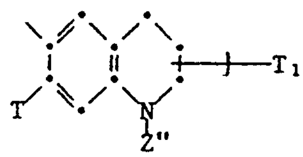

or a tetrahydroquinolinyl radical of formula

(3d) 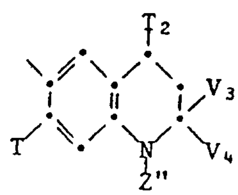

or a benzomorpholino radical of formula

(3e) 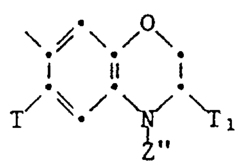

where
$m_1$ is 1 to 3,
$X_4$ and $X_5$ are independently of each other lower alkyl, cyano-lower alkyl, phenyl, benzyl, phenethyl, lower alkylphenyl or lower alkoxyphenyl, or
$X_4$ and $X_5$, together with the nitrogen atom which connects them, are pyrrolidino, piperidino or morpholino,
$X_5$ is hydrogen, halogen, lower alkyl or lower alkoxy,
Z'' is hydrogen, $C_1$-$C_8$alkyl, $C_2$-$C_6$alkoxyalkyl, $\beta$-cyanoethyl, benzyl or phenethyl,
T is hydrogen, halogen, lower alkyl, lower alkoxy, $C_1$-$C_4$acylamino or phenyl,
$T_1$ and $T_2$ are each hydrogen, halogen, hydroxyl, lower alkyl or lower alkoxy, and
$V_1$, $V_2$, $V_3$ and $V_4$ are each hydrogen, lower alkyl, $C_5$-$C_6$cycloalkyl or benzyl, or ($V_1$ and $V_2$) or ($V_3$ and $V_4$) are each together $C_4$-$C_5$alkylene, and the ring $A_1$ is unsubstituted or substituted by one or two

substituents selected from cyano, halogen, lower alkyl, phenyl and lower alkoxy.

**12.** A quinazoline compound according to claim 11, wherein in formula (3) $Y_2$ is an aminophenyl radical of formula (3a).

**13.** A quinazoline compound according to claim 12, wherein in formula (3) $X_4$ and $X_5$ are each lower alkyl or benzyl, $X_5$ is hydrogen, $R_1$ is phenyl or chlorophenyl, $Q_1$ is ethylene or propylene, $Z_1$ and $Z_2$ are oxygen, and the ring $A_1$ is unsubstituted.

**14.** A quinazoline compound according to claim 1, of formula

(4) 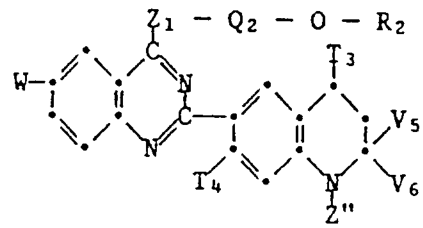

wherein
$Q_2$ is straight-chain or branched alkylene of 2 to 4 carbon atoms,
$R_2$ is phenyl, chlorophenyl, tolyl, methoxyphenyl or naphthyl,
$Z_1$ is sulfur or oxygen,
Z" is alkyl of 1 to 8 carbon atoms, $\beta$-cyanoethyl or benzyl,
$T_3$, $V_5$ and $V_6$ are each lower alkyl,
$T_4$ is hydrogen or methyl, and
W is halogen, methyl, methoxy or hydrogen.

**15.** A quinazoline compound according to claim 1, of formula

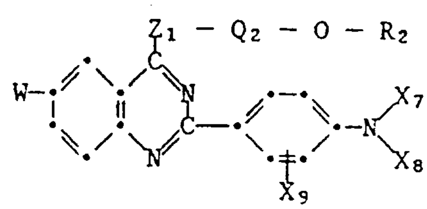

(5)

wherein
$Q_2$ is straight-chain or branched alkylene of 2 to 4 carbon atoms,
$R_2$ is phenyl, chlorophenyl, tolyl, methoxyphenyl or naphthyl,
$Z_1$ is sulfur or oxygen,
$X_7$ and $X_5$ are each lower alkyl or benzyl, or $-NX_7X_5$ is piperidino,
$X_9$ is hydrogen, methyl, methoxy or ethoxy, and
W is halogen, methyl, methoxy or hydrogen.

**16.** A quinazoline compound according to claim 15, wherein in formula (5) $Q_2$ is ethylene or propylene, $R_2$ is phenyl or chlorophenyl, $X_7$ and $X_5$ are lower alkyl, and $X_9$ and W are each hydrogen.

**17.** A process for the preparation of a quinazoline compound of the formula (1) given in claim 1, which comprises reacting an alcohol or thioalcohol compound of formula

(6) $HZ_1\text{-}Q\text{-}Z_2\text{-}R$

wherein R, Q, $Z_1$ and $Z_2$ are as defined in claim 1 with a 4-haloquinazoline compound of formula

(7) 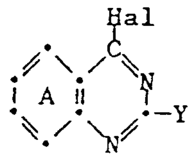

wherein A and Y are as defined in claim 1 and Hal is halogen.

**18.** The use of a quinazoline compound of a formula given in one of claims 1 to 16 as a colour former in a pressure-sensitive or heat-sensitive recording material.

**19.** A pressure-sensitive or heat-sensitive recording material which comprises in its colour-reactant system, as colour former, at least one quinazoline compound of a formula given in one of claims 1 to 16.

**20.** A pressure-sensitive recording material according to claim 19, which comprises the quinazoline compound, dissolved in an organic solvent, and at least one solid electron acceptor.

**21.** A pressure-sensitive recording material according to one of claims 19 and 20, wherein the quinazoline compound is encapsulated in microcapsules.

**22.** A pressure-sensitive recording material according to claim 21, wherein the encapsulated quinazoline compound is present in the form of a layer on the back of a transfer sheet and the electron acceptor is present in the form of a layer on the face of the receiving sheet.

**23.** A pressure-sensitive recording material according to one of claims 19 to 22, which comprises the quinazoline compound together with one or more other colour formers.

**24.** A heat-sensitive recording material according to claim 19, which comprises in at least one layer at least one colour former, one electron acceptor and if appropriate a binder, the colour former having a formula given in one of claims 1 to 16.

**Revendications**

**1.** Dérivés de quinazoline, chromogènes, de formule :

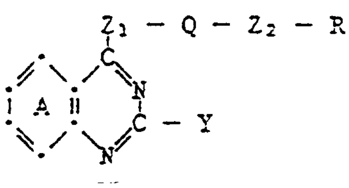 (1)

dans laquelle :
R représente un groupe phényle, biphénylyle ou naphtyle, non substitué ou substitué par un ou plusieurs substituants pris parmi les atomes d'halogène et les groupes cyano, nitro, alkyle inférieur, alcoxy inférieur, (alcoxy inférieur)carbonyle et (alkyl inférieur)carbonyle, ou un groupe thiényle, furyle, pyrrolyle, pyrazolyle, imidazolyle, pyridinyle, pyrrolidinyle, pipéridinyle, pipécolinyle, morpholinyle, thiomorpholinyle, pipérazinyle ou N-méthylpipérazinyle, non substitué ou substitué par un ou plusieurs substituants pris parmi les atomes d'halogène et les groupes cyano, alkyle inférieur, alcoxy inférieur, (alcoxy inférieur)carbonyle et noyau benzénique condensé,
$Z_1$ représente un atome d'oxygène ou de soufre,
$Z_2$ représente un atome d'oxygène ou de soufre ou un groupe

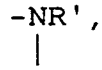

R' représente un atome d'hydrogène, un groupe alkyle ayant au plus 12 atomes de carbone, non substitué ou substitué par un ou plusieurs substituants pris parmi les atomes d'halogène et les groupes cyano et alcoxy inférieur, un groupe cycloalkyle en $C_5$-$C_6$, phényle, benzyle, phénéthyle, ou un groupe acyle ayant 1 à 12 atomes de carbone, ou bien

-NRR' représente un groupe pyrrolidinyle, pipéridinyle, pipécolinyle, morpholinyle, thiomorpholinyle, pipérazinyle ou N-méthylpipérazinyle,

Q représente un groupe alkylène ayant 2 à 8 atomes de carbone, dont la chaîne peut être interrompue par des atomes d'oxygène, des atomes de soufre ou des groupes imino -NR'-,

Y représente un reste d'aniline ou de naphtylamine, qui peut être non substitué sur le cycle carboné ou substitué sur le cycle carboné par un ou plusieurs substituants pris parmi les atomes d'halogène et les groupes hydroxyle, cyano, nitro, alkyle inférieur, alcoxy inférieur, (alcoxy inférieur)carbonyle, (alkyl en $C_1$-$C_8$)-carbonyle, alkylène en $C_3$-$C_8$, cycloalkyle en $C_5$-$C_6$, benzyle et phényle, et qui peut être non substitué sur l'atome d'azote ou mono- ou disubstitué sur l'atome d'azote par un groupe alkyle en $C_1$-$C_{12}$, alcényle en $C_2$-$C_{12}$, benzyle ou phénéthyle, éventuellement substitué par un ou plusieurs substituants pris parmi les atomes d'halogène et les groupes cyano, nitro, hydroxyle, alkyle inférieur, alcoxy inférieur et (alcoxy inférieur)-carbonyle,

ou bien Y représente un reste d'indole, de carbazole, d'indoline, de tétrahydrocarbazole, de dihydroquinoléine, de tétrahydroquinoléine, de dibenzylimide ou de benzomorpholine, qui, dans la formule (1), est lié à la quinazoline par l'intermédiaire du noyau benzénique condensé, et qui peut être non substitué sur le cycle carboné ou substitué sur le cycle carboné par un ou plusieurs substituants pris parmi les atomes d'halogène et les groupes hydroxyle, cyano, nitro, alkyle inférieur, alcoxy inférieur, (alcoxy inférieur)carbonyle, (alkyl en $C_1$-$C_8$ )carbonyle, alkylène en $C_3$-$C_6$ cycloalkyle en $C_5$-$C_6$, benzyle et phényle, et qui peut être non substitué sur l'atome d'azote ou substitué sur l'atome d'azote par un groupe alkyle en $C_1$-$C_{12}$, alcényle en $C_2$-$C_{12}$, benzyle ou phénéthyle, éventuellement substitué par un ou plusieurs substituants pris parmi les atomes d'halogène et les groupes cyano, nitro, hydroxy, alkyle inférieur, alcoxy inférieur et (alcoxy inférieur)carbonyle, et le noyau A est non substitué ou substitué par un ou plusieurs substituants pris parmi les atomes d'halogène et les groupes cyano, nitro, alkyle inférieur, alcoxy inférieur et (alcoxy inférieur)carbonyle,

le terme "inférieur" s'appliquant à des groupes ayant 1 à 5 atomes de carbone.

**2.** Dérivés de quinazoline selon la revendication 1, caractérisés en ce que, dans la formule (1), R représente un groupe phényle, biphénylyle ou naphtyle, qui est non substitué ou substitué par un ou plusieurs substituants pris parmi les atomes d'halogène et les groupes cyano, nitro, alkyle inférieur, alcoxy inférieur, (alcoxy inférieur)carbonyle et (alkyle inférieur)-carbonyle.

**3.** Dérivés de quinazoline selon la revendication 1, caractérisés en ce que, dans la formule (1), R représente un groupe phényle, chlorophényle, méthoxyphényle, tolyle ou xylyle.

**4.** Dérivés de quinazoline selon l'une quelconque des revendications 1 à 3, caractérisés en ce que, dans la formule (1), Q représente un groupe alkylène ayant 2 à 4 atomes de carbone.

**5.** Dérivés de quinazoline selon l'une quelconque des revendications 1 à 4, caractérisés en ce que, dans la formule (1), $Z_1$ et $Z_2$ représentent chacun un atome d'oxygène.

**6.** Dérivés de quinazoline selon l'une quelconque des revendications 1 à 5, caractérisés en ce que, dans la formule (1), Y représente un reste d'aniline ou de naphtylamine, mono- ou disubstitué sur l'atome d'azote par un groupe alkyle en $C_1$-$C_{12}$, alcényle en $C_2$-$C_{12}$, benzyle ou phénéthyle, non substitué ou substitué par un ou plusieurs substituants pris parmi les atomes d'halogène et les groupes cyano, nitro, hydroxyle, alkyle inférieur, alcoxy inférieur et (alcoxy inférieur)carbonyle.

**7.** Dérivés de quinazoline selon la revendication 6, caractérisés en ce que, dans la formule (1), Y représente le reste d'une aniline N,N-disubstituée.

**8.** Dérivés de quinazoline selon l'une quelconque des revendications 1 à 5, caractérisés en ce que, dans la formule (1), Y représente le reste d'une indoline, d'un tétrahydrocarbazole, d'une dihydroquinoléine, d'une tétrahydroquinoléine, d'un dibenzylimide ou d'une benzomorpholine, non substitué sur l'atome d'azote ou substitué sur l'atome d'azote.

**9.** Dérivés de quinazoline selon la revendication 8, caractérisés en ce que, dans la formule (1), Y représente un reste indolinyle, tétrahydroquinolyle ou benzomorpholino.

**10.** Dérivés de quinazoline, caractérisés en ce qu'ils répondent à la formule :

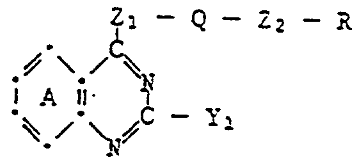

(2)

dans laquelle A, $Z_1$, $Z_2$, Q et R ont les significations indiquées dans la revendication 1, et $Y_1$ représente un groupe aminophényle de formule :

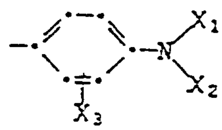

(2a)

ou un groupe hétérocyclique hydrogéné, de formule :

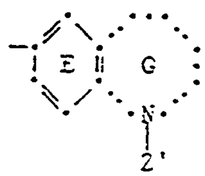

(2b)

formules dans lesquelles $X_1$ et $X_2$ représentent chacun, indépendamment l'un de l'autre, un atome d'hydrogène, un groupe alkyle ayant au plus 12 atomes de carbone, non substitué ou substitué par un ou plusieurs substituants pris parmi les atomes d'halogène et les groupes hydroxy, cyano et alcoxy inférieur, un groupe cycloalkyle en $C_5$-$C_6$,phényle, benzyle ou phénéthyle, ou un groupe phényle ou benzyle, substitué par un ou plusieurs substituants pris parmi les atomes d'halogène et les groupes nitro, cyano, alkyle inférieur, alcoxy inférieur et (alcoxy inférieur)carbonyle, ou bien
$X_1$ et $X_2$ représentent ensemble, avec l'atome d'azote qui les relie, un groupe pyrrolidinyle ou pipéridinyle, et $X_3$ représente un atome d'hydrogène ou d'halogène, ou un groupe nitro, alkyle inférieur, alcoxy inférieur ou (alcoxy inférieur)carbonyle, et
Z' représente un atome d'hydrogène ou un groupe alkyle ayant au plus 8 atomes de carbone, cycloalkyle en $C_5$-$C_6$, benzyle ou phénéthyle, non substitué ou substitué par un ou plusieurs substituants pris parmi les atomes d'halogène et les groupes cyano et alcoxy inférieur,
le noyau E est non substitué ou substitué par un ou plusieurs substituants pris parmi les atomes d'halogène et les groupes cyano, alkyle inférieur et alcoxy inférieur, et
le noyau G, pris avec le noyau E, représente un reste d'indoline, de tétrahydroquinoléine ou de benzomorpholine, qui est non substitué ou substitué une ou plusieurs fois sur les atomes de carbone par des substituants pris parmi les atomes d'halogène et les groupes cyano, hydroxyle, alkyle inférieur, alcoxy inférieur, cycloalkyle en $C_5$-$C_6$, benzyle et alkylène en $C_3$-$C_6$.

**11.** Dérivés de quinazoline selon la revendication 1, caractérisés en ce qu'ils répondent à la formule :

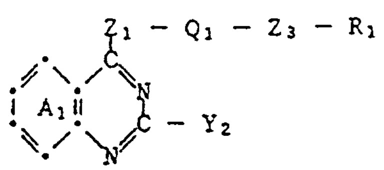

(3)

dans laquelle :
$R_1$ représente un groupe phényle ou naphtyle, éventuellement substitué par un ou plusieurs substituants pris parmi les atomes d'halogène et les groupes méthyle et méthoxy,
$Z_1$ représente un atome d'oxygène ou de soufre,
$Z_3$ représente un atome d'oxygène ou de soufre ou un groupe -NH- ou

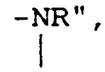

R'' représente un groupe alkyle inférieur ou bien $-NR_1R''$ représente un groupe morpholino,
$Q_1$ représente un groupe alkylène en $C_2$-$C_4$ ou le groupe $-(CH_2-CH_2-O)_{m1}-CH_2CH_2-$,
$Y_2$ représente un groupe aminophényle de formule :

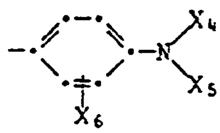

(3a)

un groupe 5-indolinyle de formule :

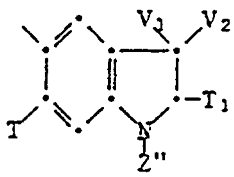

(3b)

un groupe tétrahydroquinolyle de formule :

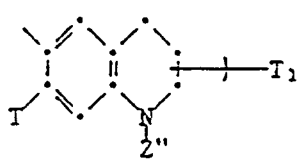

(3c)

un groupe tétrahydroquinolyle de formule :

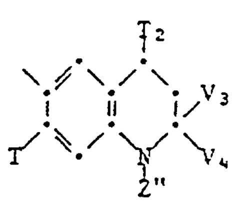

(3d)

ou un groupe benzomorpholino de formule :

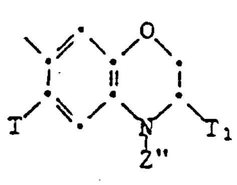

(3e)

formules dans lesquelles :

$m_1$ représente un nombre de 1 à 3,
$X_4$ et $X_5$ représentent chacun, indépendamment l'un de l'autre, un groupe alkyle inférieur, cyano(alkyle inférieur), phényle, benzyle, phénéthyle, (alkyl inférieur)phényle ou (alcoxy inférieur)phényle, ou bien
$X_4$ et $X_5$ représentent ensemble, avec l'atome d'azote qui les relie, un groupe pyrrolidino, pipéridino ou morpholino,
$X_5$ représente un atome d'hydrogène ou d'halogène, ou un groupe alkyle inférieur ou alcoxy inférieur,
Z" représente un atome d'hydrogène ou un groupe alkyle en $C_1$-$C_8$, alcoxyalkyle en $C_2$-$C_6$, $\beta$-cyanoéthyle, benzyle ou phénéthyle,
T représente un atome d'hydrogène ou d'halogène, ou un groupe alkyle inférieur, alcoxy inférieur, acylamino en $C_1$-$C_4$ ou phényle,
$T_1$ et $T_2$ représentent chacun un atome d'hydrogène ou d'halogène, ou un groupe hydroxy, alkyle inférieur ou alcoxy inférieur, et
$V_1$, $V_2$, $V_3$ et $V_4$ représentent chacun un atome d'hydrogène ou un groupe alkyle inférieur, cycloalkyle en $C_5$-$C_6$ ou benzyle, ou bien ($V_1$ et $V_2$) ou ($V_3$ et $V_4$) représentent ensemble un groupe alkylène en $C_4$-$C_5$, et
le noyau $A_1$ est non substitué ou substitué par un ou deux substituants pris parmi les atomes d'halogène et les groupes cyano, alkyle inférieur, phényle et alcoxy inférieur.

**12.** Dérivés de quinazoline selon la revendication 11, caractérisés en ce que, dans la formule (3), $Y_2$ représente un groupe aminophényle de formule (3a).

**13.** Dérivés de quinazoline selon la revendication 12, caractérisés en ce que, dans la formule (3), $X_4$ et $X_5$ représentent chacun un groupe alkyle inférieur ou benzyle, $X_5$ représente un atome d'hydrogène, $R_1$ représente un groupe phényle ou chlorophényle, $Q_1$ représente un groupe éthylène ou propylène, $Z_1$ et $Z_2$ représentent des atomes d'oxygène et le noyau $A_1$ n'est pas substitué.

**14.** Dérivés de quinazoline selon la revendication 1, caractérisés en ce qu'ils répondent à la formule :

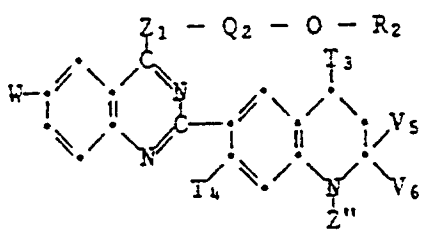

(4)

dans laquelle :
$Q_2$ représente un groupe alkylène, à chaîne droite ou ramifiée, ayant 2 à 4 atomes de carbone,
$R_2$ représente un groupe phényle, chlorophényle, tolyle, méthoxyphényle ou naphtyle,
$Z_1$ représente un atome d'oxygène ou de soufre,
Z" représente un groupe alkyle ayant 1 à 8 atomes de carbone, $\beta$-cyanoéthyle ou benzyle,
$T_3$, $V_5$ et $V_6$ représentent chacun un groupe alkyle inférieur,
$T_4$ représente un atome d'hydrogène ou un groupe méthyle, et
W représente un atome d'halogène ou d'hydrogène, ou un groupe méthyle ou méthoxy.

**15.** Dérivés de quinazoline selon la revendication 1, caractérisés en ce qu'ils répondent à la formule :

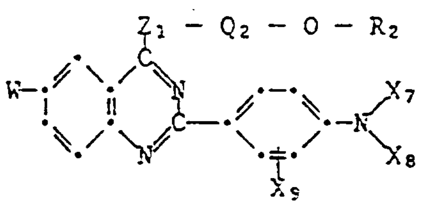

(5)

dans laquelle
$Q_2$ représente un groupe alkylène, à chaîne droite ou ramifiée, ayant 2 à 4 atomes de carbone,

$R_2$ représente un groupe phényle, chlorophényle, tolyle, méthoxyphényle ou naphtyle,
$Z_1$ représente un atome d'oxygène ou de soufre,
$X_7$ et $X_8$ représentent chacun un groupe alkyle inférieur ou benzyle, ou bien $-NX_7X_8$ représente un groupe pipéridino,
$X_9$ représente un atome d'hydrogène ou un groupe méthyle, méthoxy ou éthoxy, et
W représente un atome d'hydrogène ou d'halogène, ou un groupe méthyle ou méthoxy.

**16.** Dérivés de quinazoline selon la revendication 15, caractérisés en ce que, dans la formule (5), $Q_2$ représente un groupe éthylène ou propylène, $R_2$ représente un groupe phényle ou chlorophényle, $X_7$ et $X_8$ représentent chacun un groupe alkyle inférieur, et $X_9$ et W représentent chacun un atome d'hydrogène.

**17.** Procédé de préparation de dérivés de quinazoline répondant à la formule (1) indiquée à la revendication 1, caractérisé en ce que l'on fait réagir un alcool ou un thiol de formule :

$HZ_1$-Q-$Z_2$-R (6)

dans laquelle R, Q, $Z_1$ et $Z_2$ ont les significations indiquées à la revendication 1, avec un dérivé de quinazoline halogéné en position 4, de formule :

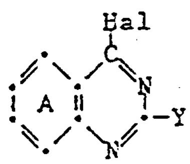

(7)

dans laquelle A et Y ont les significations indiquées à la revendication 1 et Hal représente un atome d'halogène.

**18.** Utilisation d'un dérivé de quinazoline répondant à la formule indiquée dans l'une quelconque des revendications 1 à 16, en tant qu'agent de formation de couleur, dans un produit d'enregistrement, sensible à la pression ou sensible à la chaleur.

**19.** Produit d'enregistrement, sensible à la pression ou sensible à la chaleur, caractérisé en ce qu'il contient dans son système de réactifs pour coloration, en tant qu'agent de formation de couleur, au moins un dérivé de quinazoline répondant à la formule indiquée dans l'une quelconque des revendications 1 à 16.

**20.** Produit d'enregistrement, sensible à la pression, selon la revendication 19, caractérisé en ce qu'il contient le dérivé de quinazoline, dissous dans un solvant organique, et au moins un accepteur d'électrons solide.

**21.** Produit d'enregistrement, sensible à la pression, selon la revendication 19 ou 20, caractérisé en ce que le dérivé de quinazoline est enfermé dans des microcapsules.

**22.** Produit d'enregistrement, sensible à la pression, selon la revendication 21, caractérisé en ce que le dérivé de quinazoline enfermé dans les microcapsules est présent sous la forme d'une couche au verso d'une feuille de transfert, et l'accepteur d'électrons est présent sous la forme d'une couche au recto de la feuille réceptrice.

**23.** Produit d'enregistrement, sensible à la pression, selon l'une quelconque des revendications 19 à 22, caractérisé en ce qu'il renferme le dérivé de quinazoline et un ou plusieurs autres agents de formation de couleur.

**24.** Produit d'enregistrement, sensible à la chaleur, selon la revendication 19, caractérisé en ce qu'il contient, dans au moins une couche, au moins un agent de formation de couleur, un accepteur d'électrons et éventuellement un liant, l'agent de formation de couleur ayant la formule indiquée dans

l'une quelconque des revendications 1 à 16.